# EUROPEAN PATENT APPLICATION

(11) **EP 2 745 775 A1**
(43) Date of publication of application: **25.06.2014**
(21) Application number: 12197806.8
(22) Date of filing: 18.12.2012
(51) Int. Cl.: A61B 5/083, A61B 5/087, G01F 1/684, G01N 21/64, A61B 5/08

(54) **Device for measuring respiratory gas property, airway adapter and gas analyzing unit for respiratory gas analysis**

(71) Applicant: General Electric Company, Schenectady, NY 12345 (US)
(72) Inventor: Haveri, Heikki Antti Mikael, 03150 Huhmar (FI)
(74) Representative: Illingworth-Law, William Illingworth

(57) **Abstract**

A device for measuring respiratory gas property is disclosed herein. The device includes a substrate (40) having at least two layers (49) joined on top of each other, at least one cavity (42, 51, 71, 72, 73, 74, 81, 82, 83) inside the substrate and at least one sensor (60) fixed to the substrate. The device also includes at least one signal conductor (61) connected to the sensor, and at least one signal contact (41) for communicating along the signal conductor with the sensor and for communicating outside the device. The device also includes a first protrusion (75) attached to the substrate to extend outwards from the substrate and which first protrusion can disturb the respiratory gas flow. The sensor can provide a signal indicative of respiratory gas flow rate disturbed by the protrusion. The signal conductor is disposed in the cavity inside the substrate. (Fig. 3)

## Description

### BACKGROUND OF THE INVENTION

This disclosure relates generally to a device for measuring respiratory gas property and an airway adapter. The disclosure also relates generally to gas analyzing unit for respiratory gas analysis.

During anesthesia or in critical care, patients are often mechanically ventilated instead of breathing spontaneously. The patient is connected to a patient circuit of a ventilator or an anesthesia machine by intubation: inserting an endotracheal tube to a trachea so that a gas can flow trough it into and out of a lung. The breathing circuit typically consists of the endotracheal tube, a Y-piece where inspiratory and expiratory tubes from the ventilator come together, as well as the ventilator.

It is important that the ventilation meets the patient's needs for a correct exchange and administration of oxygen (O₂), carbon dioxide (CO₂), nitrous oxide (N₂O), anesthetic agents and other gases. It is also very important to feed gases at pressures that do not damage the patient's lung. The gases should also be given at a suitable respiration rate and tidal volume. Anesthesiologists, respiratory therapists and other qualified clinicians use their professional skills to set ventilation parameters to optimally meet needs of the patient. The ventilation is often monitored with a respiratory monitor by measuring in real time concentrations of carbon dioxide, oxygen, nitrous oxide and anesthetic agent in a breathing gas. The respiratory monitors often also have spirometric measurements for monitoring a pressure and gas flow in the patient circuit. From a gas concentration and gas flow data, it is possible to calculate the patient's consumption of oxygen and production of CO₂ and furthermore for example the energy consumption and nutrition. Spirometry or the measurement of lung function, specifically the volume and/or flow of air that can be inhaled and exhaled, is also an important tool used for generating pneumotachographs, which are helpful in assessing conditions such as asthma, pulmonary fibrosis, cystic fibrosis, and COPD.

Recent developments in miniaturization of gas sensors have opened the possibility to measure all respiratory gases such as for example CO₂, N₂O, Oxygen and anesthetic agents with a mainstream gas sensor or combination of sensors, placed on an airway adapter in the patient circuit of the ventilator or an anesthesia machine. Mainstream gas sensors transform the measured concentrations to electric signals that are transmitted to the respiratory monitor. This is usually done with a cable that also feeds an operating power from the respiratory monitor to the sensor.

The spirometry has been measured conventionally for example with a sidestream gas analyzer through a pneumatic tubing connected to airway adapter positioned to patient's airways. Also an attempt has been made to combine the spirometry measurement technique known from the sidestream gas analyzers with the mainstream CO₂ gas analyzing technique. The dead space of the airway adapter is huge causing rebreathing of gases that decreases the gas exchange in the lungs. The sensor also comprises complicated pneumatic connections that connect to an analyzer further away from a patient via pneumatic tubing increasing the risk of contaminating the analyzer and increasing the breathing circuit leakage risk that decreases the gas exchange in the lungs. The sensor is also big and heavy, which increases the risk that the endotracheal tube bends and clogs preventing the breathing gas to flow into and out from the lungs suffocating the patient. In addition to size and weight the additional tubing between the airway adapter and the analyzer disturb the patient as well as the care giver. The air in the volume of the tubing compresses during the inspiration and normalizes during the expiration, which leads into back and forth gas flow in the pneumatic tubing that brings moisture into the tubing from the airways. The temperature of the tubing is lower than the air coming from the patient, which causes the moisture to condensate into the tubing, finally clogging it and degrading the measurement.

It is a difficulty to integrate different type of measurements such as respiratory gas analyzing, but especially the spirometry, into a mainstream measurement suitable system, where the analyzer is small and light and the dead space of the airway adapter low, where also the cross contamination and leakage risks have been considered. To keep the analyzer system low cost it also needs to be mass production capable. An attempt to combine a mass produced, disposable, electro-chemical gas measurement cell into the airway adapter has been made. However, the described solution does not comprise the means to measure the flow or the pressure, which are needed for example for measuring spirometry, energy consumption, nutrition etc. of a patient.

### BRIEF DESCRIPTION OF THE INVENTION

The above-mentioned shortcomings, disadvantages and problems are addressed herein which will be understood by reading and understanding the following specification.

In an embodiment a device for measuring respiratory gas property includes a substrate having at least two layers joined on top of each other, and at least one cavity inside the substrate. The device also includes at least one sensor fixed to the substrate in contact with the respiratory gas, and at least one signal conductor connected to the at least one sensor. The device also includes at least one signal contact for communicating along the at least one signal conductor with the at least one sensor and for communicating outside the device, and a first protrusion attached to the substrate to extend outwards from the substrate and which first protrusion is configured to disturb the respiratory gas flow passing the first protrusion. The sensor is configured to provide a signal indicative of respiratory gas flow rate disturbed by the protrusion.Theat least one signal conductor is configured to be disposed in the cavity inside the substrate.

In another embodiment, an airway adapter includes an airway adapter frame provided with a sampling chamber for allowing a respiratory gas flow, and a device integrated with the airway adapter frame for measuring respiratory gas property. The device includes a substrate having at least two layers joined on top of each other, and at least one cavity inside the substrate. The device also includes at least one sensor fixed to the substrate in contact with the respiratory gas in the sampling chamber, at least one signal conductor connected to the at least one sensor, and at least one signal contact for communicating along the at least one signal conductor with the at least one sensor and for communicating outside the device. The device also includes a first protrusion attached to the substrate to extend outwards from the substrate and which first protrusion is configured to disturb the respiratory gas flow passing the first protrusion. The sensor is configured to provide a signal indicative of respiratory gas flow rate disturbed by the first protrusion. The at least one signal conductor is configured to be disposed in the cavity inside the substrate.

In yet another embodiment, a gas analyzing unit for respiratory gas analysis includes an airway adapter having a sampling chamber for allowing a respiratory gas flow, and at least one optical window for an optical measurement of the respiratory gas based on infrared radiation. The gas analyzing unit also includes a device integrated with the sampling chamber for measuring respiratory gas property, the device including a substrate having at least two substrate layers joined on top of each other, and at least one cavity inside the substrate. The device also includes at least one sensor fixed to the substrate in contact with the respiratory gas in the sampling chamber, at least one signal conductor connected to the at least one sensor, and at least one signal contact for communicating along the at least one signal conductor with the at least one sensor and for communicating outside the device. The gas analyzing unit also includes a gas analyzer being detachably connectable to the airway adapter, the gas analyzer including an infrared emitter for emitting an infrared radiation through the sampling chamber, a detector for receiving the infrared radiation and providing an infrared absorption signal, and a processor for analyzing the infrared absorption signal from the detector. The device also includes a first protrusion attached to the substrate to extend outwards from the substrate, which first protrusion is configured to disturb the respiratory gas flow passing the first protrusion. The sensor is configured to provide a signal indicative of respiratory gas flow rate disturbed by the first protrusion to the processor. The at least one signal conductor is configured to be disposed in the cavity inside the substrate.

Various other features, objects, and advantages of the invention will be made apparent to those skilled in art from the accompanying drawings and detailed description thereof.

### BRIEF DESCRIPTION OF THE DRAWINGS

Figure 1 is a schematic perspective view of a gas analyzing unit incorporating an airway adapter in accordance with an embodiment in an operating environment;

Figure 2 is a schematic perspective view of an airway adapter comprising an airway adapter frame and a device for measuring respiratory gas property in accordance with an embodiment;

Figure 3 is a schematic sectional view of the device of Figure 2 for measuring respiratory gas property divided into parts; and

Figure 4 is a schematic sectional view of the device of Figure 3 for measuring respiratory gas property when assembled.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

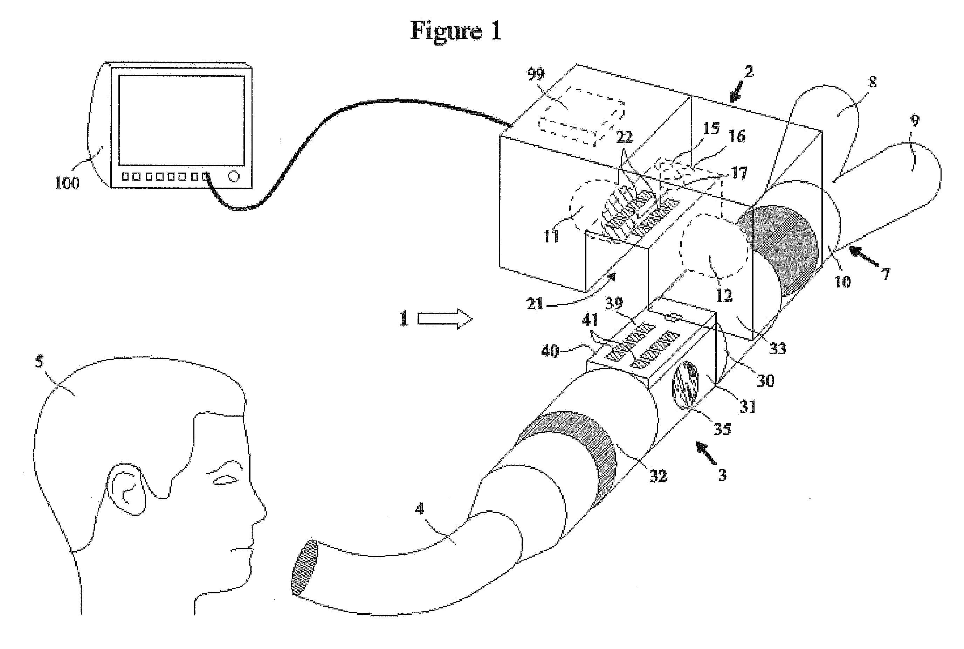

Figure 1 shows a gas analyzing unit 1 comprising a gas analyzer 2 such as a main stream gas analyzer and an airway adapter 3 in a typical operating environment where the gas analyzing unit 1 is connected through an endotracheal tube 4 to airways of a subject 5. The gas analyzing unit 1 may also be connected to a branching unit 7 such as a Y-piece having at least three limbs, one of them being an inhalation limb 8 for inspired gas, a second one being an expiration limb 9 for expired gas, a third one being a combined inspiration and expiration limb 10 for both inspired and expired gases. The inhalation limb 8 is connectable to the inspiration tube (not shown) and the expiration limb 9 is connectable to the expiration tube (not shown), which both tubes may be connected to a ventilator (not shown) for allowing the gas exchange with the airways of the subject 5. The combined inspiration and expiration limb 10 is typically connected to the airway adapter 3.

The gas analyzing unit 1 and its airway adapter 3 connectable between the endotracheal tube 4 and the branching unit 7 as shown in Figure 1 is especially designed for a mainstream measurement when a gas measurement is made directly in the main gas flow of the subject 5 and thus this can be done without taking samples out of this main gas flow. The airway adapter 3 includes an airway adapter frame 30 provided with a sampling chamber 31 between a connector 32 and a connector 33 allowing a respiratory gas flow. The connector 32, which can be a female type, and the connector 33, which can be a male type, are used to connect the airway adapter 3 as well as the gas analyzer 2 between the endotracheal tube 4 and the branching unit 7. The inner diameter of the conventional, slightly conical female connector 32 is typically 13-13.5 mm and the length 17-28 mm. The male connectors 33 may fit on male connectors in every connection of the breathing circuit, thus the inner diameter is typically approximately from 14.5 to 15.5 mm and the length 17-28 mm. The sampling chamber 31 is not limited to any special form but may be e.g. a tube or a part of tube or it may be a conventional special space with a rectangular cross sectional shape in to the direction of the gas flow. A height of a cavity inside the sampling chamber may be typically10-15 mm and a width of the cavity may be 5-15 mm. One or more optical windows 35, used for the optical gas measurement, such as gas concentration measurement or gas component measurement, based on for example absorption of an infrared radiation by different gases, are conventionally located on one side or both sides of the airway adapter 3.

The mainstream measurement analyzer construction as described above and shown in Figure 1 provides a place for small sized, low cost sensors, which can be disposed, to be located on a top of the sampling chamber 31 of airway adapter 3 according to an embodiment. However, many of the sensors are sensitive to mechanical forces such as torsion or tension, high temperature, thermal expansion, pressure, precise dimensioning, impurities etc. On the other hand the airway adapter frame 30 may comprise at least connectors 32 and 33 that may be elastic and gas tight so that they can be connected into the breathing circuit system, the endotracheal tube 4 and the branching unit 7 reliably. It is very difficult to manufacture the airway adapters 3 with so diverse properties using only one manufacturing process for example the plastic injection molding process, which however is suitable for manufacturing the airway adapter frames 30, but not suitable for sensitive sensors or their components. Consequently it is convenient to locate the sensitive sensors or the sensor components on a suitable substrate 39, which can be rigid and manufactured in an automated process according to the requirements of diverse components and sensors to form a device 39, such as a sensor board, for measurement of respiratory gas property, which property may include at least gas flow rate and possibly one of gas pressure and gas content. The airway adapter frame 30 and said device 39 are integrated together gas tightly to form an integral structure of the airway adapter 3. It is also possible to make the device 39 detachable from the adapter frame 30, but in that case the device 39 should be made suitable for example for autoclaving to prevent cross contamination between patients.

The airway adapter 3 can be placed into the coupling point 21 in the gas analyzer 2 to enable the measurement of at least one of the gas property in the breathing gas mixture, such as the gas flow rate, the gas pressure and the gas content of oxygen, carbon dioxide, volatile anesthetics or humidity. Typically the gas analyzer is detachably connectable to the airway adapter. The sensors or components located in or on the substrate 40 may be in gaseous direct or indirect contact or interaction with the breathing gases inside the airway adapter 3, whereas the gas analyzer 2 is apart from the breathing gas. Signals, such as electrical, optical, or electro-magnetic signals, proportional to measured physical magnitude of the gas are transmitted to the gas analyzer 2 through one or more cavities in the substrate 40. The airway adapter may be replaced between the patients or when the operating time is achieved to prevent cross contamination between the patients or to prevent bacteria growth. With this design the gas analyzer 2 remains clean of patient's secretions along the time and between the patients.

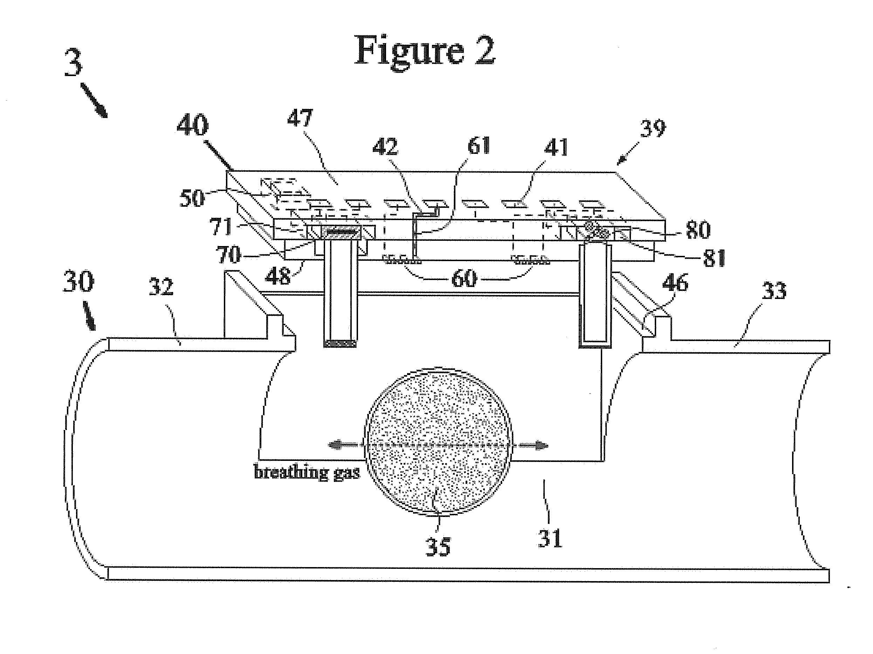

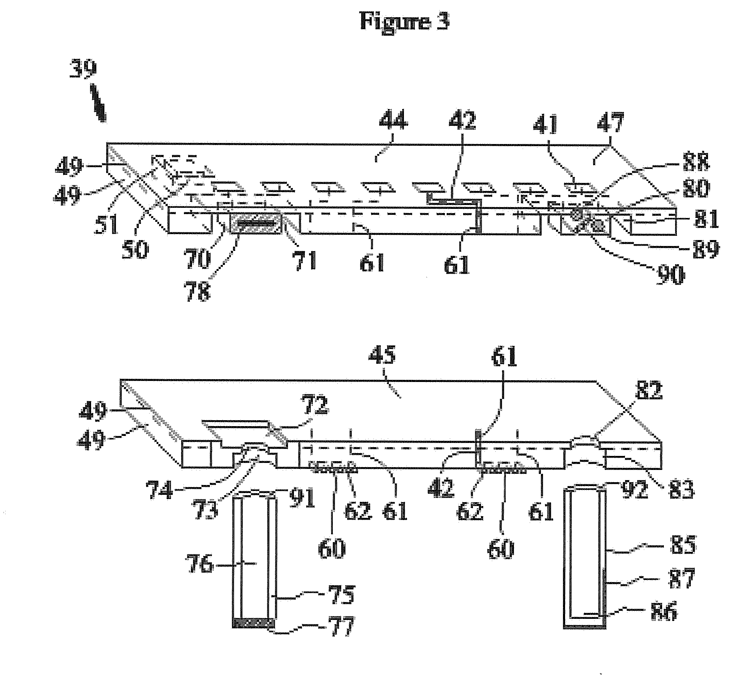

Figure 2 shows a cross sectional schematic view of the airway adapter 3 comprising the device 39 for measuring respiratory gas property, and the airway adapter frame 30 provided with the sampling chamber 31. In this specific embodiment the rectangular shaped device 39 for measuring respiratory gas property is manufactured in a separate electronics system manufacturing process, whereas the airway adapter frame 30 is manufactured with the plastics injection molding techniques. The device 39 and the airway adapter frame 30 may be integrated together to form the airway adapter 3. The substrate 40 can be made of for example thin, rectangular or orbicular shaped substrate layers 49 shown in figure 3 such as LTCC, HTCC, Alumina or FR4 or similar including at least one cavity 42 inside the substrate for a signal conductor 61, such as an electrical or optical conductor. In Figure 2 there is more than one cavity for signal conductors and also there are other cavities, such as a cavity 42, 71 and 81 for different purposes. One or more signal contacts 41, such as electrical contacts can be in the cavity or on the surface of the substrate. One or more sensors 60 or complete sensors, such as thermistors, may be placed on the surface of the substrate 40. Typically sensors, which are thermistors, are fixed on the opposite surface of the substrate compared to signal contacts 41. In Figure 2 there are other sensors, such as a sensor 70 for an absolute pressure measurement in a cavity 71 and a sensor 80 for oxygen measurement in a cavity 81. A component 50, such as a memory used to store for example sensor specific calibration information, can be located into a cavity 51 respectively, inside the substrate 40, between the substrate layers 49. All the components and sensors can be connected to signal contacts 41 along cavities 42. The specific sensors such as 60, 70 and 80 that can measure the gas properties of the breathing gas may be located on one side of the substrate 40, but preferably inside the substrate, to protect the sensitive sensors from the ambient forces, such as the user or the patient. Both the substrate with sensors and the airway adapter frame 30 can be reusable, but more advantageously they are disposable to prevent cross contamination between patients. The airway adapter frame can be made of pro-environmental or similar plastics.

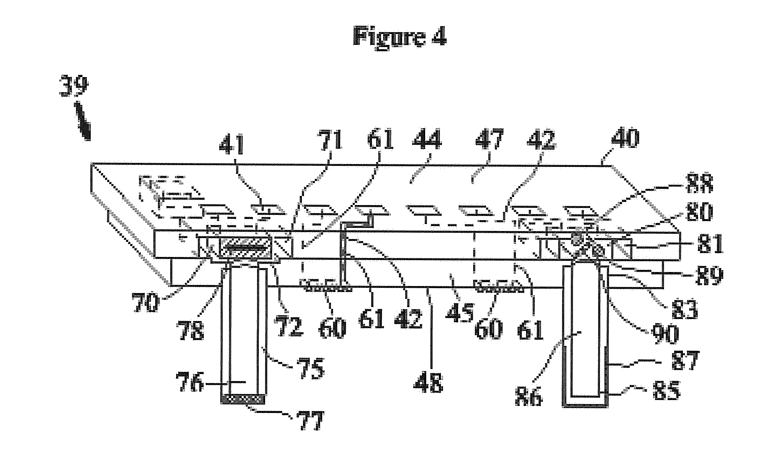

The substrate 40 is advantageous to manufacture using for example the low temperature co-fired ceramics (LTCC), but other similar techniques are also possible. The LTCC board is constructed of thin ceramic layers having one or more cavities for one or more signal conductors, such as electrical conductors, or sensors etc. between the ceramic layers. The signal conductor disposed in the cavity 42 connects with each other through via piercing the cavity through the ceramic layers. Cavities are constructed by removing parts of the ceramic layers to form a space on the surface of the substrate or inside the substrate between the layers. The cavities on the substrate's surface can be used to bury components that can be further covered with for example epoxy to protect them. The components can be buried inside the substrate also, such as the memory component 50 in figure 2. Similarly cavities can be formed through the substrate such as cavities 73, 74 for a first protrusion 75 and cavities 82, 83 for a second protrusion 85 in figures 3 and 4 or partly through the substrate such as the cavity 71 for the sensor 70 and the cavity 81 for the sensor 80. Protrusions 75 and 85 can be attached to cavities 73, 83 respectively by gluing, soldering or similar technique. Cavities can also be formed inside the substrate in to the direction of substrate layers 49, typically ceramic layers, which layers appear in figure 3, for gases and fluids to flow into the sensors or between the sensors. Ceramic material is suitable to be placed into the breathing gas flow as it is insensitive to chemicals, fluids, temperature changes etc. and does not include dissolving chemicals or material that may for example irritate the patient or the user.

The device 39 for measuring respiratory gas comprises one or more signal contacts 41, such as electrical contacts, located on one or more sides of the device 39 or its substrate 40 typically outside the airway adapter frame 30. The signal contact 41 may also be in the cavity in case its contact with the gas analyzer 2 is made possible. In the specific examples shown in figures 1, 2, 3 and 4 the signal contacts 41 are on a top surface 47 of the substrate 40 of the device 39 outside the airway adapter frame 30. The top surface 47 is away from sampling chamber 31, while a bottom surface 48 of the substrate is in contact with the respiratory gas when the device is attached to the airway adapter frame 30. One or more signal contacts 41 further connect to the sensor or different sensors and components located inside or on the substrate 40. The sensor 60 having conductor 62, which function as thermistors and are located at least on one side of the substrate 40, in this specific example on the bottom surface 48 inside the airway adapter 3, which is in connection with the breathing gases. When the airway adapter 3 is pressed into the coupling point 21 one or more signal contacts 41 connect with corresponding signal contacts 22, such as electrical contacts, in the gas analyzer 2 thus enabling the signals to be transmitted between one or more sensors and a processor 99 in the gas analyzer 2 for example or between one or more other components like the component 50 in the device 39 and for example a processor 99 in the gas analyzer 2.

The signal contacts 41, which are electrical, can be replaced with optical or radio frequency (RF) signal transmission (not shown in figures) contacts to transmit the signals from the device 39 into the gas analyzer 2 without any electrical connections between the two. However, these methods are more expensive and consume more space and electrical power. Optical signal transmission needs optical transceivers located into the device 39 or its substrate 40 and gas analyzer 2 whereas RF-transmission needs antennas, which means that at least one of the electrical contacts 41, 22 are replaced with an antenna contact. Both techniques by choice also need analog to digital signal converter into the device 39 or its substrate 40.

It is advantageous to manufacture the substrate 40 and the airway adapter frame 30 in separate manufacturing processes. The device 39 or the substrate may comprise diverse, sensitive sensors, electronic-, optical- and other components that fail easily or it may comprise cavities for electric-, pneumatic and/or optical sensors, components or fluids that may contaminate. The substrate 40 may need very special process steps that relate to for example electric board manufacturing, component assembling from different directions, soldering, gluing, coating etc. On the other hand the airway adapter frame 30 having the sampling chamber 31may comprise complicated shapes or structures, such as one or more optical windows 35 attached to a diverse, tubular frame of airway adapter 3 implemented with two or three component plastic techniques and thus may need a more complex plastics injection molds or similar method to be manufactured. It may be extremely difficult and expensive to try to combine the manufacture of plastic part with complicated electronic-, mechanical- and optical functions within one manufacturing process. Thus, after the substrate 40 and the airway adapter frame 30 have been finished in their own, separate manufacturing processes it is convenient to attach and combine the complete device 39 with the substrate 40 to a plastic molded airway adapter frame 30. The device 39 can according to an embodiment to be hermetically and gas tightly an integral part of the airway adapter frame 30 to ensure there is no breathing gas leakages through the airway adapter, which otherwise degrades the respiratory function. Hermetic connection is achieved by gluing, molding or similarly attaching the device 39 into the airway adapter frame 30. In a specific example in figure 2 the attachment can be made by gluing or similar attachment techniques through the joint 46 between the device 39 or the substrate 40 and the airway adapter frame 30. The disposability and individual use of airway adapters 3 leads into the fact that the manufacture of substrate 40 or the whole device 39 advantageously needs to be easy, fast and cost effective in large quantities. Advantageously the same applies to manufacture of the plastic airway adapter frame 30 and its attachment to the device 39.

The exploded, cross sectional view in figure 3 shows a closer view of one example of the substrate 40 of the device 39 for measuring respiratory gas processed with a technology such as LTCC. The substrate 40 is constructed of two separate portions of a top substrate portion 44 and the bottom substrate portion 45, both constructed of at least two substrate layers 49. The top substrate portion 44 and the bottom substrate portion 45 in the embodiment of figure 3 comprise cavities 71, 72, 73, 81, 82, 83 for various components, such as the first protrusion 75, the second protrusion 85, and sensors 60, 70, 80, but also one or more cavities 42 for at least one signal conductor 61 to connect the at least one sensor to at least one signal contact 41 to transmit signals further away from the device 39. The top and the bottom substrate portions 44, 45 can be firmly and hermetically attached to each other later in the manufacturing process, which is shown in a cross sectional exploded view in figure 4, when also the cavities are enclosed and the cavities for signal contacts 41 connected between the two substrate portions. With this design methodology all sensitive parts, the sensors and the components can be enclosed within the ceramic structure, such as LTCC structure, protecting them against different ambient phenomenon that may break them down.

The LTCC substrate is suitable in forming the substrate for the sensor or sensors since the ceramic material is very insensitive to chemicals, fluids, temperature changes etc. and does not include dissolving chemicals or material that may for example irritate the patient or the user. It is also very rigid material, with high Young's modulus and low thermal expansion coefficient, which minimizes all the mechanical forces such as tension and torsion directed to sensors or components attached to the substrate. Also the thermal expansion coefficient is close to silicon based electrical components, which makes it reliable in terms of thermal changes. The Young' modulus, which is a measure of stiffness for a material and describes how much torsion and tension the board can tolerate without changing its shape, is approximately 90-150 GPa. The thermal expansion coefficient, which is a measure of how much the construction expands or shrinks as the temperature increases, is 4-9 ppm/°C for LTCC depending on the LTCC material. It is advantageous that the Young' modulus for the multi-sensor board substrate is more than 20 MPa, but preferably more than 50 MPa and the best performance is achieved with value higher than 100 MPs. Similarly it is advantageous if the thermal expansion coefficient for the substrate is less than 9 ppm/°C, but preferably less than 5 ppm/°C and the best performance is achieved with a value same as for silicon 1-5 ppm/°C. Young's modulus for Alumina or HTCC is approximately 300-350 GPa whereas for commonly used FR4 based PCBs it is approximately 20-35 GPa. The thermal expansion coefficient for Alumina or HTCC is approximately 5-8 ppm/°C whereas for commonly used FR4 based PCBs it is approximately 14-20 ppm/°C in the direction of the plane (xy-axis) and approximately 95-180 ppm/°C in the direction against the plane (z-axis).

The bottom substrate portion 45 comprises two sensors 60, both located preferably symmetrically, between and in line with the first protrusions 75 and the second protrusion 85. The sensors 60 have properties that make them sensitive to thermal changes such as a cooling or heating effect caused by the air flowing past the first and second protrusions 75 and 85 and the sensors 60. Thus sensors 60 are thermistor type sensors, which connect through signal conductors 61 disposed in cavities 42 traversing through the substrate to signal contacts 41. In the particular example shown in Figure 3 and 4 the sensors 60 are constructed of devious conductors 62 having material properties sensitive to thermal changes. To increase the sensitiveness to thermal changes the sensor 60 thermal mass needs to be minimized, which is done by minimizing the sensor volume or more specifically the cross sectional area of the sensor. The width and the thickness of the sensor for measuring thermal changes is less than 50 µm, but preferably at least the thickness is less than 10 µm or as thin as possible to make the sensor as sensitive as possible to thermal changes. The sensor's sensitiveness to thermal changes also depends on the thermal conductivity of the substrate material it is placed on, since if the thermal conductivity is high the substrate function as an additional thermal mass degrading the sensitivity and the response time. The thermal conductivity for example for LTCC can be less than 2 W/mK depending on the base material, the green tape used, thus it is a suitable material to function as a substrate.

The LTCC technology is also extremely suitable for manufacturing the substrate with the sensor for measuring thermal changes having precise, small dimensions, made of materials having different properties, at low cost and in large quantities. Electrical sensors may be processed by distributing paste between the LTCC layers or on the outer surfaces of the LTCC substrate and the controlling of the thickness and the width of the sensor are easy and precise. It is also possible to trim very precise the sensor that is located on the outer surface with a laser by evaporating material away from the desired areas. To increase the sensor's sensitiveness to thermal changes the sensor can be placed on another substrate for example FR4 or HTCC, which can then be placed into the cavity in LTCC. The thermal conductivity of FR4, which is commonly used in electronic boards (PCB), is about 8 times less than that of LTCC, approximately 0.25 W/mK. Cavities and similar structures as for LTCC are also possible for FR4, but it may be more expensive to produce sensors from different materials and especially cavities, depending on the complexity of the overall design of course, since they are normally mechanically machined. Materials, such as Plyimide used in HTCC boards, has thermal conductivity about 10 times less than LTCC, approximately 0.2 W/mK and can also be used. It is also very suitable for creating for example diverse sensors, but it has other limitations when compared to LTCC. The thermal conductivity for plastics, which are good insulators, is generally between 0.2-0.5 W/mK.

The first protrusion 75 and the second protrusion 85, which second protrusion is at a distance from said first protrusion along the flow direction, function as taps that disturb the respiratory gas flow generating flow turbulences into the breathing gas flowing back and forth when passing the first and second protrusion. The breathing gas flow can carry heat energy generated by the at least one sensor 60 for measuring thermal changes, whereas the turbulences generated by the first protrusion 75 and the second protrusion 85 interfere the heat energy flow changing it into a sinusoidal type heat energy fluctuation, which frequency and amplitude are relative to the speed of gas flow. Thus the two sensors 60 located between the first protrusion 75 and the second protrusion 85 can transfer heat energy into the gas flow, but also sense the heat energy fluctuations and thus the direction of it. Two sensors 60 can also measure the transit time delay of heat energy between each other, which is also relative to the speed of breathing gas. Thus the magnitude and the frequency of turbulences generated by the protrusions are relative to the speed of the gas flowing by that can be sensed with the sensors 60 comprising conductors 62, which function as temperature sensors. With this measurement arrangement comprising a pair of protrusions and a pair of sensors the direction of the air flow can be determined also. Thermal changes are transferred into electrical signals proportional to flow rate and the direction. Furthermore the conductors 62 of the sensor 60 can also measure the absolute temperature of the breathing gas if desired, which is indicative of the patient's body temperature during expiration and the temperature of the inspired gas during inspiration.

The protrusions 75 and 85 generate turbulences into the gas flowing past them, whereas the sensors 60 generate heat into the gas flow that the gas flow carries away from the sensors. The sensors 60 can also sense the thermal changes such as the fluctuation of heat carried within the turbulent gas flow. The construction comprising two sensors 60 located between the protrusions 75 and 85 can detect the direction of the flow and measure the speed of the flow. As the cross sectional area of sampling cell 31 is known the flow rate of gas can be calculated. When the gas flows from the directions of the protrusion 75 towards the protrusion 85 the protrusion 75 generates turbulences in to the gas flow where the adjacent sensor 60 adds heat. The heated air flows to another sensor 60 by the protrusion 85 that can sense the heat fluctuation carried within the gas flow. Similarly when the gas flows from the directions of the protrusion 85 towards the protrusion 75 the protrusion 85 generates turbulences in to the gas flow where the adjacent sensor 60 adds heat. The heated air flows to another sensor 60 by the protrusion 75 that can sense the heat fluctuation carried within the gas flow. The sensitiveness of the measurement depends on the shape, diameter and the length of the protrusions 75 and 85 as well as the distances between the protrusions 75, 85 and the sensors 60. The optimal shape for the protrusions in the direction against the gas flow is circular, but other shapes are possible also. The optimum shape generates high, uniform fluctuation downstream from the protrusion, but on the other hand causes minimal restriction to gas flow.

The length of the protrusions, in the direction across the sampling chamber 31, should be long enough to ensure uniform gas flow fluctuation at the location of sensors 60, but on the other hand the length of protrusion should be as low as possible to cause minimal restriction to the gas flow and to enable additional care devices, such as a suction tube used for treating the lungs, to enter through the sampling chamber 31. The distance between the sensors 60 and protrusions 75, 85 should be longer if the diameter of protrusion is increased, since there is a non-fluctuating or undeterminable turbulent area downstream, just by the protrusions. If the distance between the sensors and the protrusions is too high the fluctuation attenuates, at least before reaching the second sensor downstream. The optimum distance between the sensors 60 and protrusions 75, 85, as well as the distance between the sensors 60, is such that with the minimum and the maximum speeds of flows desired to be measured the gas flow fluctuation at the first sensor 60 downstream from the protrusion 75 or 85 is uniform and the heat added into the gas flow by the first sensor 60 does not attenuate too much so that the second sensor 60, downstream from the protrusions 75, 85 and the first sensor 60, can sense the attenuated fluctuation, in other words the sinusoidal type heat fluctuation with amplitude and frequency, so that the speed of flow can be calculated. It is difficult to give any exact mechanical measurements for the construction described above since everything depends a lot on the overall airway adapter design as well as aimed respiration-, flow- and volume rates to be measured.

Although the first protrusion 75 primarily functions as a tap that disturb the respiratory gas flow generating flow turbulences that can be sensed with sensor or sensors 60, it can be used to measure the gas pressure as well. In the embodiment shown in figures 3 and 4 the first protrusion 75 comprise a porous membrane 77 in the end of the hollow 76 inside the first protrusion 75 to allow pressure to be conducted through the opening of the hollow 76, but to prevent liquids to enter into this hollow and the cavity 71 and to the pressure sensing element 78 of the pressure sensor 70, which is attached into the cavity 71, 72 formed when the top and bottom substrate boards are attached to each other. The first protrusion 75 in this specific embodiment can be placed with an automated pick and place machine into the bottom of the cavity 73, against the step formed by the cavity 74, where it is hermetically attached by gluing, soldering or with a similar automated attachment method, in an automated manufacturing process, to form a continuous path for the pressure to be conducted from the breathing gas inside the airway adapter frame 30 into the pressure sensing element 78. The first protrusion 75 can be made in a separate process from various materials such as plastic, ceramic, metal or similar.

The pressure sensing element 78 of the sensor 70 sense the gas pressure inside the airway adapter 3 that varies during the inspiration and expiration breathing cycles via the hollow 76 and generates an electrical signal relative to the alternating gas pressure. The sensor 70 is electrically connected to at least one signal contact 41 through signal conductor 61 disposed in the cavity 42 traversing inside the substrate to transmit the measured signal out from the airway adapter 30 as a voltage, current, resistance, capacitance etc. It is possible to construct a custom design pressure sensor inside the cavity during the LTCC process as shown in figure 3, when the benefits would be more optimized functioning, smaller size and lower cost.

The first protrusion 75 can be an integral part of the pressure sensor as well, thus a commercial pressure sensor can be used, by attaching it into the cavity 71 by soldering it from its electric connections, such as electric pins into the electrical connection inside the cavity 42 (not shown in figures). When the top and bottom substrate portions 44, 45 are attached to each other the first protrusion 75 in this case may pierce through the cavities 73, 74 in the bottom substrate portion 45 where the joint between the bottom substrate portion 45 and the first protrusion 75 is sealed with glue or similar.

The porous membrane 77 located into the end of the protrusion 75 allows the gas molecules, but prevents the liquids to enter the hollow 76 and the pressure sensor 70 preventing the functioning of the sensor 70 to degrade. The pressure drop over the membrane 77 is minimized to maximize the sensitiveness and speed to measured pressure. The pores in the porous membrane 77 should be small enough to prevent the liquids to pass, but large enough to minimize the pressure drop over the membrane. On the other hand the active surface area of the porous membrane 70 can be increased to some extent at the tip of the first protrusion 75 and it may even extend to the cylindrical sides of the first protrusion 75 if needed. However the outer surfaces of the first protrusion 75, which generate flow turbulences into the flowing air that are sensed with the conductors 62 of the sensor 60, need to have properties such as smooth and uniform faces to generate preferably a sinusoidal turbulence. It is also desirable that the first protrusion 75 is similar to the second protrusion 85 to generate similar flow turbulence to both directions of the breathing gas flow.

Although the second protrusion 85 primarily functions as a tap that disturbs the respiratory gas flow generating flow turbulences that can be sensed with sensors 60, it can be used to measure the concentration of gaseous oxygen as well. The second protrusion 85, which is shown in figures 3 and 4, may have optical properties to guide radiation and also to implement the sensor 80 for measuring the oxygen content in the breathing gas. The sensor 80, comprising an emitter 88, detector 89 and possible optical filter 90, is placed into the cavity 81 in the top substrate portion 44. The sensor 80 connects electrically to at least one signal contact 41, similar to pressure sensor 70, to transfer electrical signals from the sensor, through the cavity 42 and through the signal contact 41 out from the substrate 40 of the device 39. The second protrusion 85 can be placed with an automated pick and place machine into the cavity 83, against the step formed by the cavity 82 and firmly attached with glue or similar in automated process so that the light guide 86 such as an optical waveguide inside the protrusion 85 connects with the cavity 82 in the bottom substrate portion 45 and the cavity 81 in the top substrate portion 44 respectively. The light guide 86 can be for example a cavity inside the protrusion 86 or the light guide can be made of optical material guiding the light.

When the bottom substrate portion 45 is connected to the top substrate portion 44 the cavity 82 is brought into close contact with the sensor 80 and the cavity 82 and the light guide 86 are aligned with the emitter 88, detector 89 and the filter 90. The emitter 88 can emit radiation that is directed into the light guide 86 having a surface 87 at least partly coated with the luminophore that is excited by a radiation and which luminophore emits the luminescent radiation, indicative of oxygen concentration of the surrounding gas when the luminophore is in direct contact with the gas to confront luminophore on the walls of the second protrusion 85, to the detector 89 receiving the luminescent radiation that it transforms into an electrical signal proportional to oxygen content in the measured gas. The filter 89 may be used to direct the emitted radiation into the light guide 86 and the received radiation from the light guide 86 into the detector 89.

It is advantageous to have a separate protrusion, such as the second protrusion 85, that can be made of optically permeable materials, in a separate, clean, high quality manufacturing process, with low cost during which it can be also coated with the luminophore material. The second protrusion 85 can then be easily attached into the cavity 83 in the bottom substrate portion 45 at the end of the fully automated LTCC component assembly process.

The sensor 80 may also be located into the gas analyzer 2, more specifically into the coupling point 21, to reduce the cost of preferably a disposable airway adapter 3. In that case the second protrusion 85 extends through a cavity (not shown in figures) on the top surface 47 of top substrate portion 44 so that the light guide 86 in the second protrusion 85 can optically align and connect with the sensor 80 located into the coupling point 21 of the gas analyzer 2 when the airway adapter 3 is placed into the coupling point 21. However this design would be sensitive to dirt, thus the sensor 80 and the top surface 47 of the light guide 86 need to be covered preferably with an additional optical wall or optical window to prevent the dirt to enter the sensor 80 and the light guide 86 in case the cavity constitutes the light guide 86.

When the gas analyzer 2 as shown in figure 1 comprising an infrared emitter 11 for emitting an infrared radiation through the sampling chamber 31 and a detector 12 for receiving the infrared radiation and creating an infrared absorption signal is used for measuring the respiratory gas property, such as gas content or the gas composition of the patient, the airway adapter 3 is placed into a coupling point 21 such as a cavity of the gas analyzer 2 so that breathing gases flowing through the endotracheal tube 4 and the branching unit 7 and through sampling chamber 31 in airway adapter 3 can be analyzed by the gas analyzer 2. As the airway adapter 3 is placed into the coupling point 21 of gas analyzer 2 one or more signal contacts 41 of the device 39 connect electrically to respective at least one signal contact 22 of the coupling point 21 of the gas analyzer 2 shown in Figure 1. Through these signal contacts 22, 41 signals, such as electrical signals, proportional to the measured gas property in the gas mixture of the breathing gas is transferred for further processing in to the processor 99 of the gas analyzer 2, which may comprise an amplifier (not shown in Figure 1), an analog to digital converter (not shown in Figure 1), a memory (not shown in Figure 1) etc. to get a calculated concentration of the measured gas or to get an identification of a gas component of the respiratory gas flow. These analyzing results including this calculated concentration information and/or the analyzed gas component information, if needed, further be received by a host device 100 such as a monitor e.g. for further processing or only for displaying the information. Alternatively the electrical signal proportional to the measured gas content in the gas mixture of the breathing gas may be transferred from the device 39 to the host device 100 for further processing to get a calculated concentration of the measured gas. Also this is same with the gas composition or identification signal.

Also processing of the signal as shown in Figure 1 may be made in the processor of the airway adapter 3 or in the device 39 of the airway adapter comprising the amplifier, the analog to digital converter, the memory, the radio frequency transceiver etc. and a small battery that produces an electrical energy for the operation (not shown in figures). The processor with all these components may be integrated into the device 39, but around the airway adapter 3 as well (not shown in figures). As the airway adapter 3 and the device 39 integrated into one piece are preferably disposable rather than reusable and as the aim is to have very small sized combination of the device 39 and the airway adapter 3 as well as the low overall cost it may be more convenient to do further signal processing elsewhere. However low cost electronics comprising the processor and the radio frequency transceiver etc. as well as the small battery may be produced with a very low cost. There may be a wireless connection between the airway adapter 3 and the host device 100 for same reasons as discussed with Figure 1, but naturally the connection can be arranged through a wire, too.

The embodiments discussed hereinbefore solves many problems relating to conventional mainstream measurement of gaseous properties, such as gas concentration, gas flow and gas pressure, as well as derivatives based on the measured signals such as metabolic calculations, energy consumption etc. In the anesthesia and transportation the need for monitoring the same patient is normally hours. In intensive care the patient is usually monitored much longer time up to months, but during that period of time the breathing circuit or parts of it are often replaced several times, once every 1-3 days, as they get contaminated of mucus, bacteria, viruses etc. A shorter lifetime and a disposability of the gas sensor enable the miniaturization of conventional gas sensors to be merged into for example conventional airway adapters. This is possible for example by integrating various measurements together as has been described above.

According to the embodiments discussed hereinbefore the device 39 may be sealed hermetically into the airway adapter frame 30 to form a complete airway adapter 3, which eliminates the risk of the breathing gas leakage and the possibility of contaminating the non-disposable parts of the gas analyzer 2 thus preventing dangerous bacteria and viruses etc. to sift other patients. The disposable measurement decreases a manual and time consuming cleaning work, but most of it improves the patient safety. Furthermore using packaging techniques and mass production to make the airway adapter 3 small and inexpensive it reduces the cost of the whole measurement. Furthermore the device 39 including at least one sensor typically inside or on the bottom surface 48 of the substrate is placed directly into the breathing gas flowing inside the airway adapter 3, which decreases the response time of the gas measurement considerably. In the terms of rise time Xᵣ = Yᵣ + Zᵣ the rise time of the molecule diffusion Yᵣ is close to zero thus the rise time of the measurement Xᵣ is determined mainly by the rise time of the sensor Zᵣ, which is less than 300 ms for the new type of the sensors described in this embodiment. Similarly in the terms of fall time X_{f} = Y_{f} + Z_{f} the fall time of the molecule diffusion Y_{f} is close to zero thus the fall time of the measurement is determined mainly by the fall time of the sensor Z_{f}, which is also less than 300 ms. In the terms of the respiration rate RR it is possible to measure RR more than 60 rpm (respiration per minute), depending mainly of the response time of the sensors, which is usually enough for the most of the mechanically ventilated subjects, but also the design of the sampling cell 31. One or more signal contacts 41 of the device 39 in airway adapter 3 connect to respective one or more signal contacts 22 of the gas analyzer 2 automatically as the airway adapter 3 is pressed in to the coupling point 21 of the gas analyzer 2 thus making the device easy to use also.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A device for measuring respiratory gas property comprising:
a substrate (40) having at least two layers (49) joined on top of each other and at least one cavity (42, 51, 71, 72, 73, 74, 81, 82, 83) inside said substrate;
at least one sensor (60) fixed to said substrate in contact with the respiratory gas;
at least one signal conductor (61) connected to said at least one sensor; and
at least one signal contact (41) for communicating along said at least one signal conductor with said at least one sensor and for communicating outside said device;
**characterized in that** said device also comprising a first protrusion (75) attached to said substrate to extend outwards from said substrate and which first protrusion is configured to disturb the respiratory gas flow passing said first protrusion and that said sensor is configured to provide a signal indicative of respiratory gas flow rate disturbed by said protrusion and that said at least one signal conductor is configured to be disposed in said cavity inside said substrate.

2. The device according to claim 1, **characterized in that** said device also comprising a second protrusion (85) attached to said substrate to extend outwards from said substrate, said second protrusion having a surface (87) at least partly coated with a luminophore emitting luminescent radiation when excited by radiation, which luminescent radiation is indicative of oxygen concentration of the surrounding respiratory gas.

3. The device according to claim 2, **characterized in that** said second protrusion is provided with optical properties to guide radiation.

4. The device according to claim 3, **characterized in that** said substrate comprises also a cavity (81) which is provided with a sensor (80) having an emitter (88) configured to emit radiation to said second protrusion.

5. The device according to claim 4, **characterized in that** said sensor having said emitter also comprising a detector (89) for receiving luminescent radiation from said second protrusion and an optical filter (90) to direct the emitted radiation into said second protrusion and to direct said received luminescent radiation from said second protrusion into said detector.

6. The device according to claim 1, **characterized in that** said substrate also comprises a cavity (71), which is provided with an absolute pressure sensor (70).

7. The device according to claim 6, **characterized in that** said first protrusion configured to disturb the respiratory gas flow is provided with a hollow (76) inside said first protrusion providing pneumatic communication with the respiratory gas.

8. The device according to claim 7, **characterized in that** said cavity with the absolute pressure sensor is in direct or indirect contact with said hollow inside said first protrusion.

9. The device according to claim 7, **characterized in that** said hollow is provided with a membrane to prevent liquids to enter said hollow inside said first protrusion.

10. The device according to claim 1, **characterized in that** said substrate comprises at least two portions, which are a top substrate portion (44) and a bottom substrate portion (45), both portions comprising said at least two layers joined on top of each other, said portions leaving therebetween one or more cavities when joined together.

11. The device according to claim 1, **characterized in that** said sensor (60) is a thermistor on a bottom surface (48) of said substrate, which bottom surface is configured to be in contact with the respiratory gas.

12. The device according to claim 2, **characterized in that** said second protrusion having a surface at least partly coated with a luminophore is also configured to disturb the respiratory gas flow passing said second protrusion, and which second protrusion is at a distance from said first protrusion along the flow direction.

13. The device according to claim 12, **characterized in that** at least two sensors (60) is provided between said first and second protrusion to provide signals indicative of inspiration and expiration gas flow rate, which flows are to opposite directions.

14. An airway adapter comprising:
an airway adapter frame (30) provided with a sampling chamber (31) for allowing a respiratory gas flow; and
a device (39) integrated with said airway adapter frame for measuring respiratory gas property, said device comprising a substrate (40) having at least two layers (49) joined on top of each other and at least one cavity (42, 51, 71, 72, 73, 74, 81, 82, 83) inside said substrate, at least one sensor (60) fixed to said substrate in contact with the respiratory gas in said sampling chamber, at least one signal conductor (61) connected to said at least one sensor, and at least one signal contact (41) for communicating along said at least one signal conductor with said at least one sensor and for communicating outside said device;
**characterized in that** said device also comprising a first protrusion (75) attached to said substrate to extend outwards from said substrate and
which first protrusion is configured to disturb the respiratory gas flow passing said first protrusion and that said sensor is configured to provide a signal indicative of respiratory gas flow rate disturbed by said first protrusion and that said at least one signal conductor is configured to be disposed in said cavity inside said substrate.

15. A gas analyzing unit for respiratory gas analysis comprising:
an airway adapter (3) having a sampling chamber (31) for allowing a respiratory gas flow and at least one optical window (35) for an optical measurement of the respiratory gas based on infrared radiation;
a device (39) integrated with said sampling chamber for measuring respiratory gas property, said device comprising a substrate (40) having at least two substrate layers (49) joined on top of each other and at least one cavity (42, 51, 71, 72, 73, 74, 81, 82, 83) inside said substrate, at least one sensor (60) fixed to said substrate in contact with the respiratory gas in said sampling chamber, at least one signal conductor (61) connected to said at least one sensor, and at least one signal contact (41) for communicating along said at least one signal conductor with said at least one sensor and for communicating outside said device; and
a gas analyzer (2) being detachably connectable to said airway adapter, said gas analyzer (2) comprising , an infrared emitter (11) for emitting an infrared radiation through said sampling chamber, a detector (12) for receiving the infrared radiation and providing an infrared absorption signal and a processor (99) for analyzing said infrared absorption signal from said detector,
**characterized in that** said device also comprising a first protrusion (75) attached to said substrate to extend outwards from said substrate, which first protrusion is configured to disturb the respiratory gas flow passing said first protrusion and that said sensor is configured to provide a signal indicative of respiratory gas flow rate disturbed by said first protrusion to said processor and that said at least one signal conductor is configured to be disposed in said cavity inside said substrate.
